(19) Europäisches Patentamt
European Patent Office
Office européen des brevets

(11) Publication number: **0 160 430**
**B1**

(12) **EUROPEAN PATENT SPECIFICATION**

(45) Date of publication of patent specification: **14.11.90**

(51) Int. Cl.$^5$: **A 61 K 7/48**

(21) Application number: **85302480.0**

(22) Date of filing: **09.04.85**

(54) **Skin treatment composition.**

(30) Priority: **11.04.84 GB 8409401**

(43) Date of publication of application:
**06.11.85 Bulletin 85/45**

(45) Publication of the grant of the patent:
**14.11.90 Bulletin 90/46**

(84) Designated Contracting States:
**AT BE CH DE FR GB IT LI NL SE**

(56) References cited:
**EP-A-0 007 785**
**EP-A-0 028 853**
**EP-A-0 048 153**
**DE-A-2 940 909**
**US-A-4 122 029**
**US-A-4 197 316**
**US-A-4 311 695**
**US-A-4 385 049**
**US-A-4 423 041**

**The component fatty acids of oils and fats, Second Edition, 1952, pages 65 and 141**

(73) Proprietor: **UNILEVER PLC**
**Unilever House Blackfriars P.O. Box 68**
**London EC4P 4BQ (GB)**
(84) **GB**

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE AT**

(72) Inventor: **Benzoni, André Jean Edouard**
**11 Boulevard Marx-Dormoy**
**F-93190 Livry-Gargan (FR)**

(74) Representative: **Tonge, Robert James et al**
**UNILEVER PLC Patents Division P.O. Box 68**
**Unilever House**
**London EC4P 4BQ (GB)**

**EP 0 160 430 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to emulsions suitable for topical application to human skin. More particularly, the invention is concerned with water-in-oil emulsions which can be applied to the skin to improve its general condition, and to prevent, alleviate or cure skin disorders such as dry or chapped skin or clinical conditions such as psoriasis or ichthiosis.

A soft, supple and flexible skin has a marked cosmetic appeal and is an attribute of normal functioning epidermis. The outer layer of the epidermis, that is the stratum corneum, can however become dry and flaky following exposure to adverse climatic conditions, or excessive contact with detergents or solvents which can result in the loss of skin moisture, with the result that the skin loses its soft, supple and flexible characteristics. Emollients such as fats, phospholipids and sterols have in the past been used to soften dry skin, but it is apparent that these emollients are only partially effective as a remedy for this type of condition.

Also, topical application of the skin of classical humectants is unlikely to alleviate this problem since they are not particularly skin substantive and are generally rinsed from the skin during washing.

Water-in-oil emulsions have been employed in the formulation of cosmetic and pharmaceutical ointments, emollients creams, lotions and the like. However, while such emulsions can impart desirable characteristics of water-repellancy to the skin and provide a means whereby fats, oils and waxes can be absorbed onto human skin, the use of such emulsions in skin treatment products has sometimes been precluded because they have been implicated in toxicity, skin irritancy or excessive greasiness in use. The problem of instability during storage can also occur, particularly with high internal phase cosmetic and pharmaceutical water-in-oil emulsions.

It should be explained that by "high internal phase emulsion" is meant an emulsion in which the volume of the internal phase occupies at least 75% of the total volume of the emulsion.

It has been proposed in EP—A—0 048 153 (Unilever) to provide a high internal phase water-in-oil emulsion comprising a branched chain non-polar oil, a nonionic liquid emulsifier having an HLB value of from 1 to 7, a soluble salt of magnesium, and a product produced by reacting sodium magnesium fluorolithosilicate trioctahedral montmorillonite clay with a quaternary ammonium salt.

It has also been proposed in US Patent No. 4 385 049 (K-V Pharmaceutical Co.) to provide emulsions for topical application in which the internal phase is non-lipoidal and comprises at least 75% of the emulsion, and the external phase is lipoidal. The lipoidal phase can comprise vegetable oils such as sunflower seed oil and an emulsifier such as polyglycerol oleate or glycerol monoisostearate having at least two hydrogen bonding sites per molecule. The technical examples illustrate the incorporation of Silicone Fluid 200 (100 $mm^2s^{-1}$), a non-volatile polydimethyl siloxane.

Other prior proposals include those of Yu & van Scott USP 4 197 316 which describes products for the treatment of dry skin which including hydroxy alkanoic acids having up to 6 carbon atoms in the molecule, such acids being buffered with triethanolamine, and Unilever EP—A 7785 which discloses skin treatment compositions containing 2-hydroxyoctanoic acid in which the pH is adjusted to values of up to 3.5 by addition of sodium hydroxide. Neither of these latter proposals suggests the use of an emulsion, especially a high internal phase emulsion, as a vehicle for the active ingredients indicated.

It is therefore evident that there exists a need for a simple, effective treatment for skin which is dry or flaky to improve its general condition, and which can also alleviate or prevent the development of disorders which gave rise to dry or flaky skin.

It has now be discovered that dry, flaky or chapped skin can be treated successfully to restore it to its normal soft, supple and flexible condition, by topical application of a shelf stable, relatively non-greasy high internal phase water-in-oil emulsion that in the oily phase comprises an oil whose fatty acid components comprise a very high level of essential fatty acid.

Accordingly, the invention provides a high internal phase water-in-oil emulsion comprising an oily phase and an aqueous phase, in which the oily phase comprises:

(a) from 1 to 3% by weight of a vegetable oil whose fatty acid components comprise at least 55% by weight of essential fatty acid;

(b) from 5 to 25% by weight of a silicone oil ingredient comprising a dispersion in a volatile silicone of a polymer of dimethyl polysiloxane with polyoxyethylene and/or polyoxypropylene side chains having a molecular weight of from 10,000 to 50,000 and having the structure:

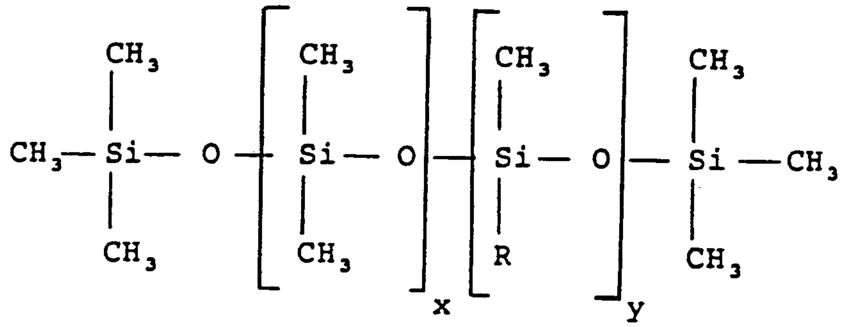

where R is - 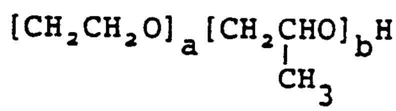

a has a value of from 9 to 115,
b has a value of from 0 to 50,
x has a value of from 133 to 673,
y has a value of from 25 to 0.25, and
(c) from 0.5 to 10% by weight of a nonionic liquid emulsifier having an HLB value of from 1 to 7.

The emulsion according to the invention consists of an internal phase which is aqueous and an external phases which is oily. Preferably, the emulsion is a high internal phase water-in-oil emulsion comprising from 75 to 98% by volume of an aqueous phase and from 2 to 25% by volume of an oily phase.

The vegetable oil which forms an ingredient of the emulsion is one whose fatty acid components comprise at least 55% by weight of essential fatty acid.

Preferably the fatty acid components of the oil comprise at least 60%,, most preferably at least 70% by weight of essential fatty acid.

Usually the major proportion of essential fatty acid component comprises linoleic acid.

Examples of suitable vegetable oils for use in the emulsions according to the invention are given below, together with their respective mean essential fatty acid contents. Normally, the essential fatty acid will be present in the oil as a mono-, di- or triglyceride ester.

| Vegetable seed oil | Essential fatty acid % |
|---|---|
| thistle (*C. tinctorius*) | 76 |
| saffron thistle | 70 |
| safflower | 67 |
| thistle (*C. carduncalus*) | 65 |
| grape | 60 |
| linseed | 72 |
| poppy (*P. somniferum*) | 62 |
| sunflower | 59 |
| hemp | 84 |
| soya bean | 63 |

It is to be understood that the above list is a selection of the preferred oils that can be employed and that the invention is not limited solely to the use of these oils. One or a mixture of two or more of such oils can be employed.

The quantity of the specially selected oil or oils, the fatty acid components of which comprise a substantial proportion of essential fatty acid, will normally form from 1 to 3%, preferably 1 to 2%, by weight of the emulsions.

If more than 3% by weight of such oils are employed, then the high internal phase emulsions can be

difficult to prepare and can exhibit poor stability during storage at temperatures between 20°C and 55°C, which comprise the normal temperature range to which the emulsions according to the invention are likely to be exposed during storage in factory, warehouse, shop or home. If less than 1% by weight of such oils is employed, the benefits attributable to the presence of a substantial proportion of essential fatty acid in such oils is unlikely to provide a noticeable skin benefit when the emulsion is applied topically to the skin.

The silicone oil ingredient which forms an ingredient of the emulsion comprises a 10% dispersion of a polymer of dimethyl polysiloxane with polyoxyethylene and/or polyoxypropylene side chains having the structure:

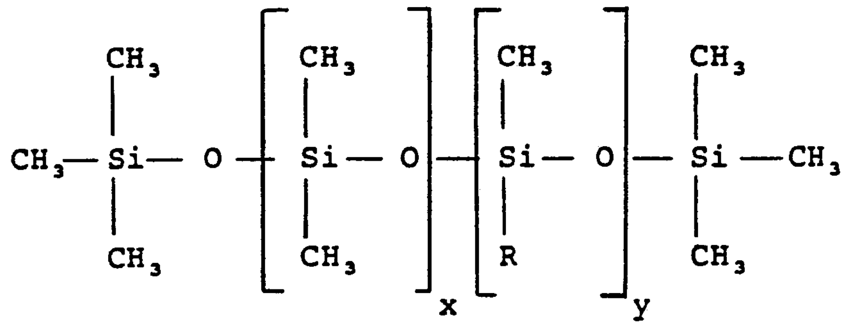

where R is - 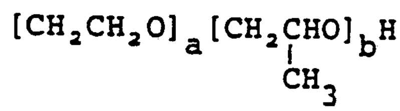

a has a value of from 9 to 115,
b has a value of from 0 to 50,
x has a value of from 133 to 673,
y has a value of from 25 to 0.25.

Preferably, the dimethyl polysiloxane polymer is one in which:
a has a value of from 10 to 114,
b has a value of from 0 to 49,
x has a value of from 388 to 402,
y has a value of from 15 to 0.75.
the group R having a molecular weight of from 100 to 5000.

A particularly preferred dimethyl polysiloxane polymer is one in which:
a has a value 14
b has a value 13
x has a value 249
y has a value 1.25.

The dimethyl polysiloxane polymer is usually provided as a dispersion in a volatile siloxane. The silicone oil ingredient accordingly can comprise from 1 to 20% by volume of the polymer and from 80 to 99% by volume of the volatile siloxane. Ideally, the silicone oil ingredient consists of a 10% dispersion of the polymer in the volatile siloxane.

Examples of the volatile siloxanes include volatile polydimethyl cyclosiloxane, such as one having a viscosity of less than 5 $mm^2sec^{-1}$, for example DOW CORNING 344 Fluid, and volatile hexamethyldisiloxane having a viscosity of not more than 0.65 $mm^2sec^{-1}$, for example DOW CORNING 200 Fluid (0.65 $mm^2s^{-1}$).

A particularly preferred silicone oil ingredient is cyclomethicone and dimethicone copolyol, such as DOW CORNING Q2-3225C Formulation Aid. (DOW CORNING is a trade mark).

The emulsion can also, optionally, comprise a non-volatile silicone oil such as polydimethylsiloxane copolymer having a viscosity in excess of 5 $mm^2s^{-1}$, such as that having a viscosity of from 100 to 350 $mm^2s^{-1}$, for example, DOW CORNING 200® Fluid (100 $mm^2s^{-1}$ or higher).

The emulsion according to the invention will normally comprise from 5 to 25%, preferably from 8 to 20% and ideally from 10 to 18% by weight of the silicone oil ingredient.

The emulsifier which also forms an ingredient of the emulsion according to the invention will normally be a nonionic liquid emulsifier having a HLB value of from 1 to 7. Preferably the emulsifier will have an HLB value of from 2 to 6.

Examples of suitable emulsifiers are:

| | HLB Value |
|---|---|
| ARLACEL® 987 (sorbitan isostearate) by Atlas | 4.3 |
| HOSTAPHAT® KO 300N (moni-, di-, and triphosphoric esters of oleic acid) by Hoechst | 2.3 |
| IMWITOR® K (glycerol monoisostearate) by Witco | 3.7 |
| BRIJ® 92 (polyoxyethylene(2)oleyl ether) by Atlas | 4.9 |
| Triglycerol monooleate by PVO International | 4.0 |
| ARLACEL® 80 (sorbitan monooleate) by Atlas | 4.3 |
| ARLACEL® 83 (sorbitan sesquioleate) by Atlas | 3.7 |
| ARLACEL® 85 (sorbitan trioleate) by Atlas | 1.8 |
| Decaglycerol tetraoleate by PVO International | 6.0 |
| Decaglycerol octaoleate by PVO International | 4.0 |
| AMEROX® or SIMULSOL® 2 (polyethoxylated(2)oleyl alcohol) by Produits Chimiques de la Montagne Noire | 6.7 |
| HOECHST 2721 (polyglycerol sequioleate) | 4.0 |

The quantity of emulsifier in the emulsion is from 0.5 to 10%, preferably 1 to 5% by weight of the emulsion.

If the emulsion contains less than 0.5% of emulsifier, it is unlikely that the emulsion, if obtained, will remain stable on storage, whereas if the emulsion contains more than 10% of emulsifier, it is no longer possible to obtain a higher internal phase emulsion, and also, the stability of the emulsion is unlikely to be further improved.

The emulsion also comprises water. The quantity of water in the emulsion is from 0.1 to 97.9%, preferably 1 to 97, most preferably 60 to 95% by weight of the emulsion.

If the emulsion contains more than 97.9% of water, the stability of the emulsion on storage is likely to be poor and syneresis can occur.

The emulsion according to the invention can also optionally comprise other ingredients which are generally cosmetically acceptable and which do not detract from the stability and efficacy of the emulsion. Such ingredients include emollients, solvents, humectants, thickeners, moisturisers, antioxidants, surfactants, anti-inflammatory agents, healing agents, preservatives, buffering agents, antiseptics, antibacterial compounds, antibiotics, germicides, keratolytic agents, abradants, perfumes or skin colouring as use in face make up preparations.

When the emulsion according to the invention is likely to be exposed to the air such that any volatile ingredients, such as silicones that may be present, are likely to be lost by evaporation, it is advantageous to include in the formulation of the emulsion finely divided silica, such as AEROSIL® 812, in order to reduce such evaporative losses.

The above examples of other ingredients is not intended to be exhaustive and many others can be employed. Further examples are given in McCutcheon's "Functional Materials" 1976 Annual published by M C Publishing Co., New Jersey.

Generally, the amount of each of the above other ingredients which optionally can be employed will be that recommended by the suppliers or manufacturers or that which is conventionally employed in the art, and which will not detrimentally affect the nature and functions of the emulsion.

The invention also provides a process for the preparation of a water-in-oil high internal phase emulsion suitable for topical application to the skin, which process comprises the steps of:

(i) forming an aqueous phase comprising water and water-soluble ingredients;

(ii) forming an oily phase comprising the silicone oil ingredient, the emulsifier and other oil-soluble ingredients except perfume;

(iii) adding the aqueous phase gradually to the oily phase at 30° to 50°C, preferably at about 40°C, with vigorous stirring to form an emulsion;

(iv) and finally adding perfume together with other volatile ingredients other than the silicone oil ingredient.

The emulsion according to the invention will normally take the form of an opaque cream, white in colour unless deliberately coloured by means of added colouring matter, which can be applied topically with the finger or with a suitable applicator to the affected area of skin.

Repeated application, say twice daily, to dry, flaky or chapped skin will usually be sufficient to eliminate or at least reduce the severity of the condition.

The emulsion will normally be packaged in a suitable container such as a compressible tube or lidded jar or pot.

The invention is illustrated by the following examples:

Examples 1 to 3

Three high internal phase water-in-oil emulsions according to the invention were prepared from the following ingredients:

| Ingredient | % w/w 1 | % w/w 2 | % w/w 3 |
|---|---|---|---|
| *Vegetable oil* | | | |
| Saffron thistle seed oil | 2 | 2 | — |
| Thistle (*Cartamus tinctorius*) seed oil | — | — | 2 |
| *Silicone oil ingredient* | | | |
| DOW CORNING® Q2 3225C Formulation Aid | 10 | 10 | 10 |
| *Other silicone ingredients* | | | |
| DOW CORNING® 344 fluid | 5 | 4 | — |
| DOW CORNING® 200 fluid (100 $mm^2s^{-1}$) | 2 | 2 | 2 |
| DOW CORNING® 200 fluid (5 $mm^2s^{-1}$) | — | — | 4 |
| *Emulsifier* | | | |
| Polyglyceryl-2-sesquiisostearate | 2 | 2 | 2 |
| *Other ingredients* | | | |
| other emollients | 6 | 8 | 10 |
| silica (AEROSIL® 812) | 2 | 2 | 2 |
| sodium glutamate | 3 | 3 | 3 |
| preservative (water soluble) | 0.5 | 0.5 | 0.5 |
| anti-oxidant, perfume (oil-soluble) | 1.5 | 1.5 | 1.5 |
| water | 66 | 65 | 63 |
| | 100 | 100 | 100 |
| | % v/v | | |
| oily phase | 22.5 | 23.5 | 24.5 |
| aqueous phase | 77.5 | 76.5 | 75.5 |

The emulsions were packaged in air-tight screw-topped jars and each was shown to be completely stable in that the syneresis did not occur, even after six months' storage at temperatures of up to 42°C.

When applied topically to dry, chapped or detergent-damages skin, each emulsion was found to be more effective in improving the condition of the skin than similar emulsions which did not contain either a vegetable oil containing a very high level of essential fatty acid or a silicone oil ingredient as herein defined, such as DOW CORNING® Formulation Aid Q2 3225C.

Examples 4 & 5

Two further water-in-oil emulsions according to the invention were prepared from the following ingredients:

| Ingredient | % w/w 4 | % w/w 5 |
|---|---|---|
| *Vegetable oil* | | |
| Safflower seed oil | 1.5 | — |
| Thistle (*Cartamus carduncalus*) seed oil | — | 2.5 |
| *Silicone oil ingredient* | | |
| DOW CORNING® Q2 3225C Formulation Aid | 8 | 12 |
| *Other silicone ingredients* | | |
| DOW CORNING® 344 fluid | 4 | — |
| DOW CORNING® 200 fluid (100 $mm^2s^{-1}$) | — | 4 |
| DOW CORNING® 200 fluid (5 $mm^2s^{-1}$) | 2 | 2 |
| *Emulsifier* | | |
| HOSTAPHAT® KO 300N | 3 | — |
| IMWITOR® 780 K | — | 2 |
| *Other ingredients* | | |
| Other emollients | 5 | 5 |
| BENTONE® 38 | 0.5 | 0.5 |
| preservative (water-soluble) | 0.5 | 0.5 |
| antioxidant, perfume (oil soluble) | 1.5 | 1.5 |
| water | 74 | 70 |
| | 100 | 100 |
| oily phase | 21 | 24 |
| aqueous phase | 79 | 76 |

Examples 6 & 7

Two further water-in-oil emulsions according to the invention were prepared from the following ingredients:

| Ingredient | % w/w 6 | % w/w 7 |
|---|---|---|
| *Vegetable oil* | | |
| Grape seed oil | 2 | — |
| Linseed oil | — | 1 |
| *Silicone oil ingredient* | | |
| DOW CORNING® Q2 3225C Formulation Aid | 6 | 3 |
| *Other silicone ingredients* | | |
| DOW CORNING® 344 fluid | 3 | — |
| DOW CORNING® 200 fluid (100 $mm^2s^{-1}$) | — | 3 |
| DOW CORNING® 200 fluid (5 $mm^2s^{-1}$) | 1 | 1 |
| *Emulsifier* | | |
| BRIJ® 92 | 4 | — |
| Triglycerol monooleate | — | 5 |

| Ingredient | % w/w 6 | 7 |
|---|---|---|
| *Other ingredients* | | |
| Other emollients | 7 | 6 |
| Magnesium sulphate | 0.3 | 0.5 |
| preservative (water-soluble) | 0.5 | 0.5 |
| antioxidant, perfume (oil soluble) | 1.5 | 1.5 |
| water | 74.7 | 78.5 |
| | 100 | 100 |
| | % v/v | |
| oily phase | 20.5 | 16.5 |
| aqueous phase | 79.5 | 83.5 |

Examples 8 & 9

Two further water-in-oil emulsions according to the invention were prepared from the following ingredients:

| Ingredient | % w/w 8 | 9 |
|---|---|---|
| *Vegetable oil* | | |
| Poppy (*P. somniferum*) seed oil | 2.5 | — |
| Sunflower seed oil | — | 2 |
| *Silicone oil ingredient* | | |
| DOW CORNING® Q2 3225C Formulation Aid | 10 | 10 |
| *Other silicone ingredients* | | |
| DOW CORNING® 344 fluid | 5 | — |
| DOW CORNING® 200 fluid (100 $mm^2s^{-1}$) | 2 | 2 |
| DOW CORNING® 200 fluid (5 $mm^2s^{-1}$) | — | 4 |
| *Emulsifier* | | |
| ARLACEL® 80 | 1.5 | — |
| ARLACEL® 83 | — | 3.5 |
| *Other ingredients* | | |
| Other emollients | 4 | 5 |
| Sodium glutamate | 3 | 3 |
| preservative (water-soluble) | 0.5 | 0.5 |
| antioxidant, perfume (oil soluble) | 1.5 | 1.5 |
| water | 70 | 68.5 |
| | 100 | 100 |
| | % v/v | |
| oily phase | 23.5 | 25 |
| aqueous phase | 76.5 | 75 |

Examples 10 & 11

Two further water-in-oil emulsions according to the invention were prepared from the following ingredients:

| Ingredient | % w/w 10 | 11 |
|---|---|---|
| *Vegetable oil* | | |
| Hemp | 1.5 | — |
| Soya bean | — | 2.5 |
| *Silicone oil ingredient* | | |
| DOW CORNING® Q2 3225C Formulation Aid | 12 | 13 |
| *Other silicone ingredients* | | |
| DOW CORNING® 344 fluid | 1 | 2 |
| DOW CORNING® 200 fluid (100 $mm^2s^{-1}$) | 2 | — |
| DOW CORNING® 200 fluid (5 $mm^2s^{-1}$) | 3 | — |
| *Emulsifier* | | |
| Decaglycerol octaoleate | 3 | — |
| HOECHST 2721 | — | 5 |
| *Other ingredients* | | |
| Other emollients | 8 | 13 |
| Sodium glutamate | 3 | 3 |
| preservative (water-soluble) | 0.5 | 0.5 |
| antioxidant, perfume (oil soluble) | 1.5 | 1.5 |
| water | 64.5 | 59.5 |
| | 100 | 100 |
| | % v/v | |
| oily phase | 29 | 37 |
| aqueous phase | 77 | 76 |

The emulsions according to Examples 4 to 11 can similarly be packaged in airtight containers and will remain stable on storage.

When applied topically to dry, chapped or detergent-damaged skin, each emulsion will be effective in improving the condition of the skin.

**Claims**

1. A high internal phase water-in-oil emulsion, comprising an oily phase and an aqueous phase which is suitable for topical application to human skin, in which the oily phase of the emulsion comprises:

(a) from 1 to 3% by weight of a vegetable oil whose fatty acid components comprise at least 55% by weight of essential fatty acid;

(b) from 5 to 25% by weight of a silicone oil ingredient comprising a dispersion in a volatile silicone of a polymer of dimethyl polysiloxane with polyoxypropylene and/or polyoxyethylene side chains, the polymer having a molecular weight of from 10,000 to 50,000 and having the structure:

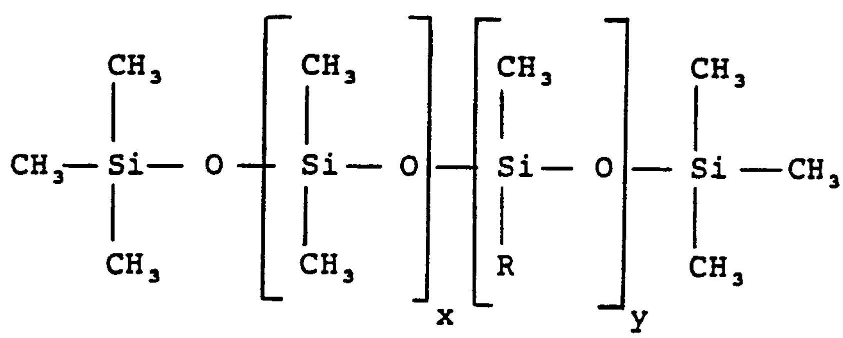

where R is $-[CH_2CH_2O]_a[CH_2\underset{CH_3}{\underset{|}{C}}HO]_bH$

a has a value of from 9 to 115,
b has a value of from 0 to 50,
x has a value of from 133 to 673,
y has a value of from 25 to 0.25, and
(c) from 0.5 to 10% by weight of a nonionic liquid emulsifier having an HLB value of from 1 to 7.

2. An emulsion according to claim 1 in which the oily phase forms from 2 to 25% by volume and the aqueous phase forms from 75 to 98% by volume of the emulsion.

3. An emulsion according to claim 1 or 2, in which the fatty acid components of the oil comprise at least 60% by weight of essential fatty acid.

4. An emulsion according to claim 1, 2 or 3, in which the fatty acid components of the oil comprise at least 70% by weight of essential fatty acid.

5. An emulsion according to any preceding claim, in which a major proportion of the essential fatty acid comprises linoleic acid.

6. An emulsion according to any preceding claim, in which the oil is chosen from the seed oil derived from thistle (*C. tinctorius*), safron thistle, thistle (*C. carduncalus*), grape, linseed, poppy (*P. somniferum*), sunflower, hemp, soya bean or mixtures thereof.

7. An emulsion according to any preceding claim, in which the dimethyl polysiloxane polymer is one in which:
a has a value of from 10 to 114,
b has a value of from 0 to 49,
x has a value of from 388 to 402,
y has a value of from 15 to 0.75,
the group R having a molecular weight of from 1000 to 5000.

8. An emulsion according to any preceding claim, in which the dimethyl polysiloxane polymer is one in which:
a has the value 14,
b has the value 13,
x has the value 249,
y has the value 1.25.

9. An emulsion according to any preceding claim in which the dimethyl polysiloxane polymer forms from 8 to 20% by weight of the emulsion.

10. An emulsion according to any preceding claim in which the emulsifier has an HLB value of from 2 to 6.

11. An emulsion according to any preceding claim which additionally comprises a perfume.

12. An emulsion according to any preceding claim in which the water forms from 0.1 to 97.9% by weight of the emulsion.

13. An emulsion according to any preceding claim in which the water forms from 60 to 95% by weight of the emulsion.

14. An emulsion according to any preceding claim in which the oily phase comprise:
(a) from 1 to 3% by weight of a vegetable oil whose fatty acid components comprise at least 60% by weight of linoleic acid;
(b) from 5 to 25% by weight of a silicone oil ingredient comprising a dispersion in a volatile silicone of a polymer of dimethyl polysiloxane with polyoxyethylene and polyoxypropylene side chains, the polymer having a molecular weight of from 10,000 to 50,000 and having the structure:

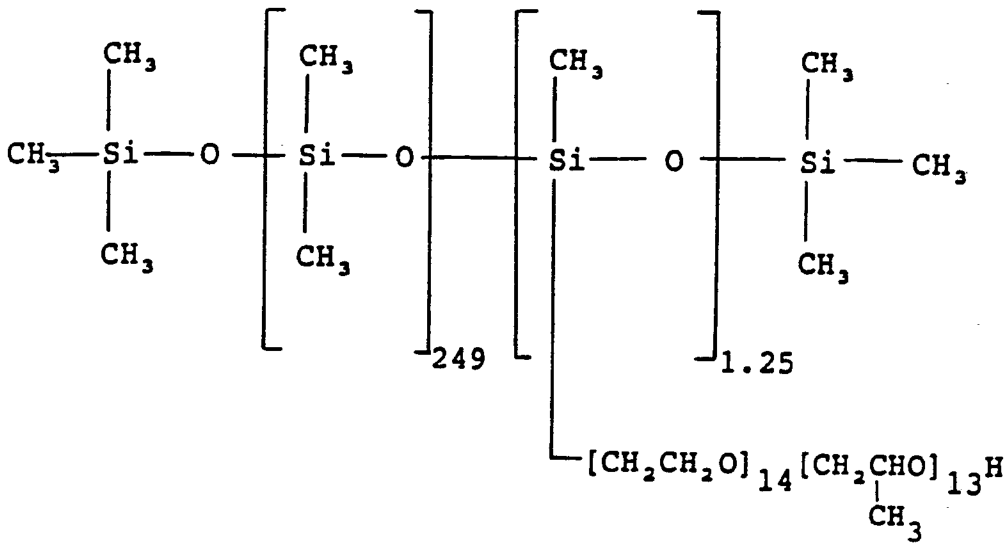

and

(c) from 0.5 to 10% by weight of a nonionic liquid emulsifier having an HLB value of from 1 to 7.

15. A process for the preparation of a water-in-oil high internal phase emulsion suitable for topical application to the skin, according to any preceding claim, which process comprises:

(i) forming an aqueous phase comprising water and water-soluble ingredients;

(ii) forming an oily phase comprising the silicone oil ingredient, the emulsifier and other oil-soluble ingredients except perfume;

(iii) adding the aqueous phase gradually to the oily phase at 30° to 50°C, preferably at about 40°C, with vigorous stirring to form an emulsion;

(iv) and finally adding perfume together with other volatile ingredients other than the silicone oil ingredient.

**Patentansprüche**

1. Eine mit hoher-interner Phase versehene Wasser-in-Öl Emulsion, umfassend eine ölige Phase und eine wässrige Phase, die zur topischen Anwendung auf menschlicher Haut geeignet ist und bei der die Ölige Phase der Emulsion:

(a) 1 bis 3 Gew.-% eines pflanzlichen Öls, dessen Fettsäure-Komponenten wenigstens 55 Gew.-% essentielle Fettsäure umfassen;

(b) 5 bis 25 Gew.-% eines Siliconöl-Bestandteile, umfassend eine Dispersion in einem flüchtigen Silicon eines Dimethylpolysiloxanpolymers mit Polyoxypropylen- und/oder Polyoxyethylen-Seitenketten, wobei das Polymer ein Molekulargewicht von 10000 bis 50000 und die Struktur:

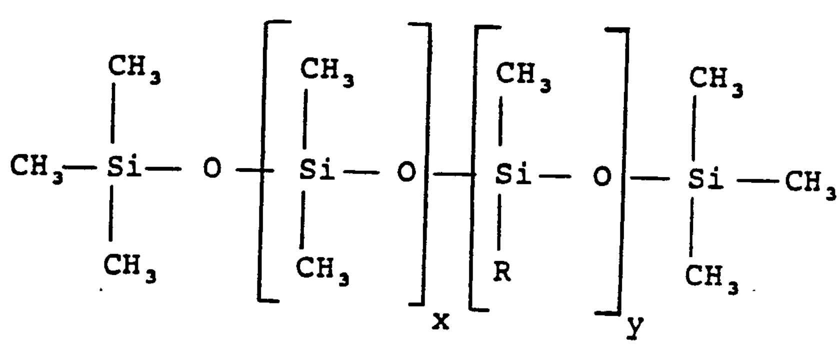

aufweist worin R -

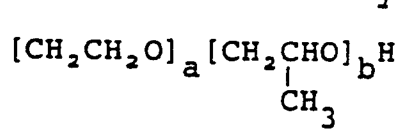

ist,

a einen Wert von 9 bis 11 aufweist,

b einen Wert von 0 bis 50 aufweist,

x einen Wert von 133 bis 673 aufweist,

y einen Wert von 25 bis 0,25 aufweist und

(c) 0,5 bis 10 Gew.-% eines nicht-ionischen, flüssigen Emulgators mit einem HLB-Wert von 1 bis 7 umfaßt.

2. Emulsion nach Anspruch 1, worin die ölige Phase 2 bis 25 Vol.-% und die wässrige Phase 75 bis 98 Vol.-% der Emulsion bildet.

3. Emulsion nach Anspruch 1 oder 2, worin die Fettsäure-Komponenten des Öls wenigstens 60 Gew.-% essentielle Fettsäure umfassen.

4. Emulsion nach Anspruch 1, 2 oder 3, worin die Fettsäure-Komponenten des Öls wenigstens 70 Gew.-% essentielle Fettsäure umfassen.

5. Emulsion nach einem der vorhergehenden Ansprüche, worin ein Großteil der essentiellen Fettsäuren Linolsäure umfaßt.

6. Emulsion nach einem der vorhergehenden Ansprüche, worin das Öl ausgewählt ist unter Samenöl der Distel (*C. tinctorius*), Safran-Distel, Distel (*C. carduncalus*), Weinbeere, Leinsamen, Mohn (*P. somniferum*), Sonnenblume, Hanf, Soyabohne oder Mischungen davon.

7. Emulsion nach einem der vorhergehenden Ansprüche, worin das Dimethylpolysiloxanpolymer ein solches ist, bei dem

a einen Wert von 10 bis 114 aufweist,
b einen Wert von 0 bis 49 aufweist,
x einen Wert von 388 bis 402 aufweist,
y einen Wert von 15 bis 0,75 aufweist.

wobei die Gruppe R ein Molekulargewicht von 1000 bis 5000 aufweist.

8. Emulsion nach einem der vorhergehenden Ansprüche, worin das Dimethylpolysiloxanpolymer ein solches ist, bei dem

a den Wert 14 aufweist,
b den Wert 13 aufweist,
x den Wert 249 aufweist,
y den Wert 1,25 aufweist.

9. Emulsion nach einem der vorhergehenden Ansprüche, worin das Dimethylpolysiloxanpolymer 8 bis 20 Gew.-% der Emulsion bildet.

10. Emulsion nach einem der vorhergehenden Ansprüche, worin der Emulgator einen HLB-Wert von 2 bis 6 aufweist.

11. Emulsion nach einem der vorhergehenden Ansprüche, die zusätzlich ein Parfüm enthält.

12. Emulsion nach einem der vorhergehenden Ansprüche, worin das Wasser 0,1 bis 97,9 Gew.-% der Emulsion bildet.

13. Emulsion nach einem der vorhergehenden Ansprüche, worin das Wasser 60 bis 95 Gew.-% der Emulsion bildet.

14. Emulsion nach einem der vorhergehenden Ansprüche, worin die ölige Phase:

(a) 1 bis 3 Gew.-% eines pflanzlichen Öls, dessen Fettsäure-Komponenten wenigstens 60 Gew.-% Linolsäure umfassen;

(b) 5 bis 25 Gew.-% eines Siliconöl-Bestandteils, umfassend eine Dispersion in einem flüchtigen Silicon eines Dimethylpolysiloxanpolymers mit Polyoxypropylen- und/oder Polyoxyethylen-Seitenketten, wobei das Polymer ein Molekulargewicht von 10000 bis 50000 und die Struktur:

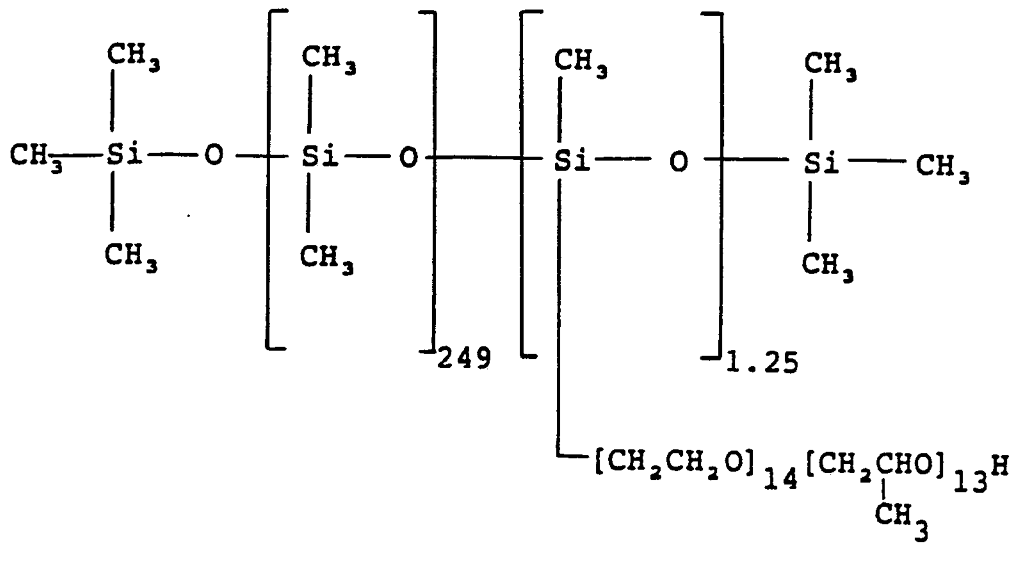

aufweist
und

(c) 0,5 bis 10 Gew.-% eines nicht-ionischen, flüssigen Emulgators mit einem HLB-Wert von 1 bis 7 umfaßt.

15. Verfahren zur Herstellung einer Wasser-in-Öl Emulsion mit hoher-interner Phase, die für die topische Anwendung auf der Haut geeignet ist, gemäß einem der vorhergehenden Ansprüche, umfassend:

(i) Bilden einer wässrigen Phase, umfassend Wasser und wasserlösliche Bestandteile;

(ii) Bilden einer öligen Phase, umfassend den Siliconöl-Bestandteil, den Emulgator und andere öllösliche Bestandteile außer Parfüm;

(iii) stufenweise Zufügen der wässrigen Phase zu der öligen Phase bei 30 bis 50°C, vorzugsweise bei etwa 40°C, unter kräftigem Rühren, um die Emulsion zu bilden.

(iv) und schließlich Zufügen von Parfume zusammen mit anderen flüchtigen Bestandteilen, außer dem Siliconöl-Bestandteil.

**Revendications**

1. Emulsion eau dans l'huile à forte teneur en phase interne comprenant une phase huileuse et une phase aqueuse, qui convient pour l'application locale sur la peau, dans laquelle la phase huileuse de l'émulsion comprend:

(a) de 1 à 3% en poids d'une huile végétale dont les constituants acides gras comprennent au moins 55% en poids d'acide gras essentiel;

(b) de 5 à 25% en poids d'un ingrédient huile de silicone comprenant une dispersion dans une silicone volatile d'un polymère de diméthylpolysiloxane avec des chaînes latérales polyoxyéthylène et/ou polyoxypropylène, le polymère ayant une masse moléculaire de 10 000 à 50 000 et ayant pour structure:

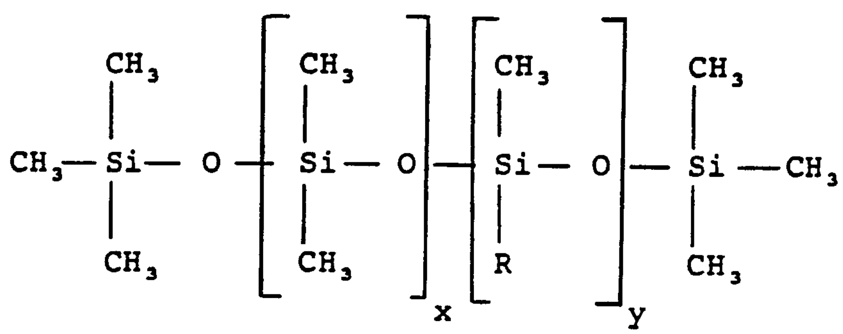

dans laquelle R est - 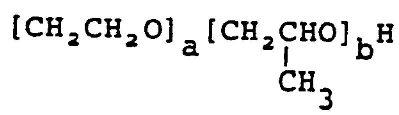

a a une valeur de 9 à 115,
b a une valeur de 0 à 50,
x a une valeur de 133 à 673,
y a une valeur de 25 à 0,25, et

(c) de 0,5 à 10% en poids d'un émulsifiant liquide non ionique ayant un rapport hydrolipophile de 1 à 7.

2. Emulsion selon la revendication 1, dans laquelle la phase huileuse forme de 2 à 25% en volume et la phase aqueuse forme de 75 à 98% en volume de l'émulsion.

3. Emulsion selon la revendication 1 ou 2, dans laquelle les constituants acides gras de l'huile comprennent au moins 60% en poids d'acide gras essentiel.

4. Emulsion selon la revendication 1, 2 ou 3, dans laquelle les constituants acides gras de l'huile comprennent au moins 70% en poids d'acide gras essentiel.

5. Emulsion selon l'une quelconque des revendications précédentes, dans laquelle une proportion majeure de l'acide gras essentiel comprend l'acide linoléique.

6. Emulsion selon l'une quelconque des revendications précédentes, dans laquelle l'huile est choisie parmi l'huile de graine dérivée du chardon (*C. tinctorius*), du carthame, du chardon (*C. carduncalus*), du raisin, du lin, du pavot (*P. somniferum*), du tournesol, du chanvre, du soja ou de leurs mélanges.

7. Emulsion selon l'une quelconque des revendications précédentes, dans laquelle le polymère diméthylpolysiloxane est un polymère dans lequel:

a a une valeur de 10 à 114,
b a une valeur de 0 à 49,
x a une valeur de 388 à 402,
y a une valeur de 15 à 0,75

le groupe R ayant une masse moléculaire de 1000 à 5000.

8. Emulsion selon l'une quelconque des revendications précédentes, dans laquelle le polymère diméthylpolysiloxane est un polymère dans lequel:

a a la valeur 14
b a la valeur 13
x a la valeur 249
y a la valeur 1,25.

9. Emulsion selon l'une quelconque des revendications précédentes, dans laquelle le polymère diméthylpolysiloxane forme de 8 à 20% en poids de l'émulsion.

10. Emulsion selon l'une quelconque des revendications précédentes, dans laquelle l'émulsifiant a un rapport hydrolipophile de 2 à 6.

11. Emulsion selon l'une quelconque des revendications précédentes, qui comprend en outre un parfum.

12. Emulsion selon l'une quelconque des revendications précédentes, dans laquelle l'eau forme de 0,1 à 97,9% en poids de l'émulsion.

13. Emulsion selon l'une quelconque des revendications précédentes, dans laquelle l'eau forme de 60 à 95% en poids de l'émulsion.

14. Emulsion selon l'une quelconque des revendications précédentes, dans laquelle la phase huileuse comprend:

(a) de 1 à 3% en poids d'une huile végétale dont les constituants acides gras comprennent au moins 60% en poids d'acide gras linoléique;

(b) de 5 à 25% en poids d'un ingrédient huile de silicone comprenant une dispersion dans une silicone volatile d'un polymère de diméthylpolysiloxane avec des chaînes latérales polyoxyéthylène et/ou polyoxypropylène, le polymère ayant une masse moléculaire de 10 000 à 50 000 et ayant pour structure:

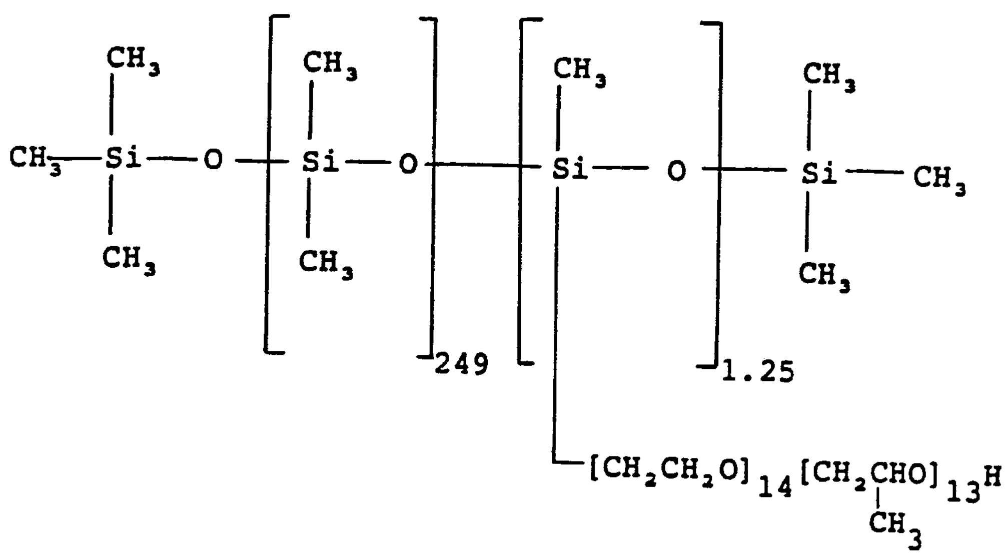

et

(c) de 0,5 à 10% en poids d'un émulsifiant liquide non ionique ayant un rapport hydrolipophile de 1 à 7.

15. Procédé de préparation d'une émulsion eau dans l'huile à forte teneur en phase interne convenant à l'application locale sur la peau, selon l'une quelconque des revendications précédentes, ce procédé comprenant les étapes qui consistent à:

(i) former une phase aqueuse comprenant de l'eau et des ingrédients hydrosolubles;

(ii) former une phase huileuse comprenent l'ingrédient huile de silicone, l'émulsifiant les autres ingrédients oléosolubles, à l'exception du parfum;

(iii) ajouter progressivement la phase aqueuse à la phase huileuse à 30°—50°C, de préférence à environ 40°C, sous agitation vigoureuse, pour former une émulsion;

(iv) et enfin ajouter le parfum ainsi que les autres ingrédients volatils autres que l'ingrédient huile de silicone.